# EUROPEAN PATENT APPLICATION

(11) **EP 1 775 296 A1**
(43) Date of publication of application: **18.04.2007**
(21) Application number: 05765126.7
(22) Date of filing: 24.06.2005
(51) Int. Cl.: C07D 471/08

(54) **ALCOHOL OXIDATION CATALYST AND METHOD OF SYNTHESIZING THE SAME**

(30) Priority: 25.06.2004 JP 2004187321; 28.03.2005 JP 2005091433
(71) Applicant: TOHOKU UNIVERSITY, Aoba-ku Sendai-shi, Miyagi 980-8577 (JP)
(72) Inventor: IWABUCHI, Yoshiharu, TOHOKU UNIVERSITY, Sendai-shi, Miyagi 980-8577 (JP); SHIBUYA, Masatoshi, TOHOKU UNIVERSITY, Sendai-shi, Miyagi 980-8577 (JP); TOMIZAWA, Masaki, TOHOKU UNIVERSITY, Sendai-shi, Miyagi 980-8577 (JP)
(74) Representative: Seiffert, Klaus
(86) International application number: PCT/JP2005/011646
(87) International publication number: WO 2006/001387

(57) **Abstract**

An organic oxidation catalyst for alcohols which is environmentally less harmful and with which efficient oxidation can be conducted. The oxidation catalyst for alcohols is a 1-alkyl-2-azadamantan-N-oxyl which has a nitroxyl group incorporated in the adamantane skeleton and was synthesized from as a base material a bicyclic compound obtained by the Grob-type ring-opening reaction of 1,3-adamantanediol. Due to the nitroxyl group on the adamantane skeleton, the α-position hydrogen is stabilized based on Bredt's rule and the stability of the oxoammonium group generated by the oxidation thereof is ensured. Compared to TEMPO, which is a conventional oxidation catalyst, this catalyst is reduced in steric hindrance and is usable in a wide range of reaction fields. Because of this, not only a primary alcohol but a secondary alcohol having a sterically complicated structure, which has been difficult to oxidize with TEMPO, can be oxidized at a high efficiency.

## Description

### FIELD OF THE INVENTION

The present invention relates to an organic oxidation catalyst for the oxidation of alcohols, in particular such an organic catalyst that is very ecofriendly, and to a method of synthesizing the same. More particularly, the invention relates to the technology of nitroxyl radical-based selective oxidation of alcohols to aldehydes, ketones and carboxylic acids.

### BACKGROUND OF THE INVENTION

Aldehydes, ketones, carboxylic acids, and derivatives thereof (carbonyl compounds) constitute one of the most important constructing block families in synthetic organic chemistry. A comprehensive compilation of methods for preparing them has made primary alcohols and secondary alcohols ideal starting materials for producing aldehydes, ketones and carboxylic acids. The oxidation of alcohols to carbonyl compounds is one of the most fundamental reactions in organic synthesis, and a number of excellent oxidizing agents and oxidation methods have so far been developed. In the art, the oxidation reactions of alcohols are carried out with oxidizing agents based on heavy metals such as transition metals. The conventional oxidizing agents include heavy metal reagents, such as chromium(VI) compounds and ruthenium, manganese and vanadium compounds, peroxides, activated dimethyl sulfoxide (DMSO), and hypervalent iodine compounds.

Since, however, it is a matter of concern that heavy metals such as transition metals may exert harmful influences on the environment and, further, since the oxidation reactions of alcohols are of importance, it has been desired that the efficiency of oxidation reactions of alcohols be further increased and the environment-friendliness thereof be improved. A disadvantageous feature of many oxidation reactions is that they are relatively hard to perform or the reagents are difficult to prepare or handle. In particular, heavy metal-containing reagents are in most cases highly toxic and very harmful to the environment. This is critically important when they are intended for industrial use.

In recent years, 2,2,6,6-tetramethylpiperidin-N-oxyl (hereinafter referred to also as "TEMPO") has become widely utilized as an oxidation catalyst for alcohols in lieu of the conventional heavy metal-based oxidizing agents with the experiment described in Non-Patent Document 1 as a turning point. The reaction mechanisms concerned are shown in Fig. 2. TEMPO is said to be an organic oxidizing agent of the low environmental load type as compared with heavy metals. In the initial stage of development, however, it was used together with such an organic cooxidizer as mCPBA. Thereafter, NaOC1, which is inexpensive, simple and easy to handle and, further, of the low environmental load type, was found to be an excellent cooxidizer, and TEMPO has since been used in combination with such cooxidizers. When, however, TEMPO is used as an oxidizing agent, those compounds which have an olefinic bond within the molecule undergo decomposition; therefore, attempts have been made to use various cooxidizers other than NaOC1 in combination with TEMPO, as shown in Non-Patent Document 2 and Non-Patent Document 3, for instance.
[Patent Document 1] Japanese Kokai (Laid-Open) Publication No. 2000-95862 (JP, A, 2000-95862)
[Patent Document 2] Japanese Patent Application No. H06-69190
[Non-Patent Document 1] Golubev V. A. et al.: Izv. Akad. Nauk SSSR, Ser. Khim. 1965, p. 1927
[Non-Patent Document 2] Lidia D. L., et al.: J. Org. Chem. 2003, vol. 68, p. 4999
[Non-Patent Document 3] Miller R. A., et al.: Org. Lett. 2003, vol. 53, p. 285

### DISCLOSURE OF INVENTION

### PROBLEMS TO BE SOLVED BY THE INVENTION

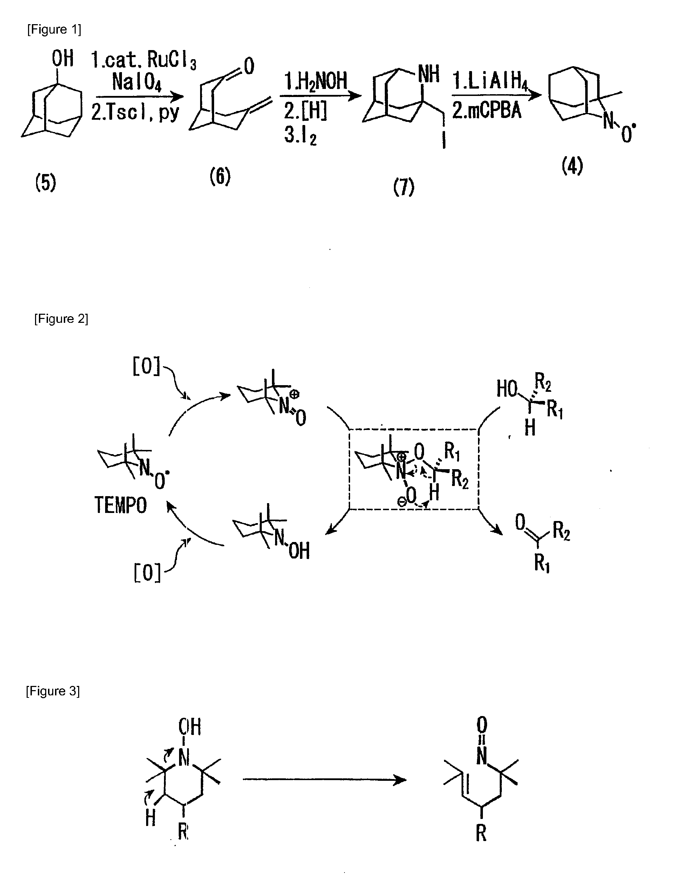

However, TEMPO widely utilized as an alcohol oxidation catalyst still has some problems. Although it is an excellent primary-selective oxidation catalyst for substrates in which a primary hydroxyl group and a secondary hydroxyl group coexist, TEMPO is not effective in oxidizing secondary alcohols having a sterically more complicated structure, hence is limited in catalyst performance. For example, TEMPO has a weak point in that the reaction yield markedly decreases in the case of secondary alcohols having a bulky steric structure. Further, a stability problem caused by the chemical structure of TEMPO is that it readily undergoes such decomposition as shown in Fig. 3 due to the chemical structure thereof.

It is an object of the present invention to solve the above-discussed prior art problems and propose an organic oxidation catalyst for alcohols which is rich in stability from the chemical structure viewpoint and excellent in environment-friendliness and enables efficient oxidation as well as a method of synthesizing the same.

The present inventors have made extensive investigations in order to accomplish the above object. As a result, the inventors have paid attention to the oxoammonium ion species known as active species in oxidation. The inventors have then succeeded in realizing that an oxoammonium group should be incorporated into the adamantane skeleton. The inventors have also succeeded in realizing the following: it can be expected that by doing so, the hydrogen in the α-position be stabilized according to the Bredt's rule, the stability of the oxoammonium group be ensured, the steric hindrance in the vicinity of the active center be reduced as compared with TEMPO, a wide reaction field can be secured, the catalytic oxidation of alcohols having a sterically complicated structure can be carried out efficiently and, at the same time, a high level of stability from the chemical structure viewpoint can be provided owing to the adamantane skeleton.

The present inventors have succeeded in synthesizing 1-alkyl-2-azaadamantan-N-oxyl species having a nitroxyl group (N-oxyl group) incorporated in the adamantane skeleton, in particular 1-methyl-2-azaadamantan-N-oxyl (hereinafter referred to also as "1-methyl-AZADO"), starting with 1-adamantanol. This compound has been found to serve as an organic catalyst low in environmental impacts, showing a higher catalytic turnover number for primary alcohols as compared with TEMPO, and further capable of also highly efficiently oxidizing secondary alcohols having a sterically complicated structure, which can hardly be oxidized with TEMPO. Furthermore, the inventors have succeeded in developing a technology of improving the operability and efficiency of synthetic methods and thereby improving overall yields.

The present invention has been completed based on the above-mentioned findings and as a result of further investigations. Thus, the gist of the invention consists in the following:
(1) An azaadamantan-N-oxyl compound of the formula (1) : 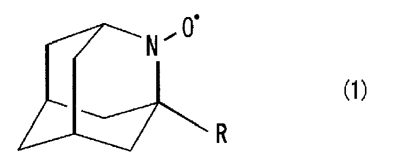 wherein R is an alkyl group, or a derivative thereof.
(2) A method of producing an azaadamantan-N-oxyl compound of the formula (1) given above, or a derivative thereof, which comprises at least the step of oxidizing an azaadamantane compound of the formula (2): 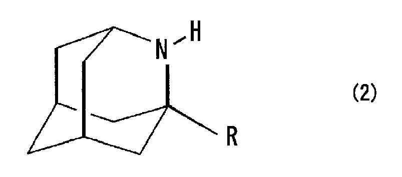 wherein R is an alkyl group, or a derivative thereof.
(3) A method of producing an azaadamantan-N-oxyl compound of the formula (1) given above, or a derivative thereof, which comprises at least the steps of
   subjecting a bicyclo[3.3.1]nonanyl-3-amine compound of the formula (3): 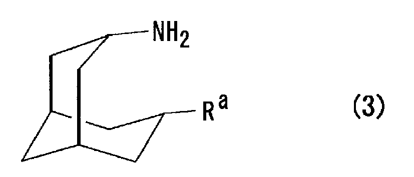 wherein R^{a} is an alkyl group or an alkylidene group bound to the bicyclo ring via a double bond, or a derivative thereof, to intramolecular ring closing reaction to form an azaadamantane ring and
   oxidizing the thus-obtained azaadamantane compound (2) or a derivative thereof.
(4) A method of producing an azaadamantan-N-oxyl compound of the formula (1) given above, or a derivative thereof, which comprises at least a process selected from the group consisting of the first process consisting of steps: converting a bicyclo compound obtained by the Grob type ring opening reaction of 1,3-adamanatnediol to a corresponding oxime, reducing the resultant oxime to a corresponding amine, and treating the amine with iodine to form a corresponding iodinated compound and the second process consisting of steps: deiodination of the iodinated compound, hydrogenation or alkylation and then oxidation of the resulting compound.
(5) A method of producing an azaadamantan-N-oxyl compound of the formula (1) given above, or a derivative thereof, which comprises at least a process selected from the group consisting of the first process consisting of steps: converting a bicyclo compound obtained by the Grob type ring opening reaction of 1,3-adamanatnediol to a corresponding oxime, reducing the resultant oxime to a corresponding amine, and further treating the amine with a carbamating (carbamate forming) agent to form a corresponding carbamate compound and the second process consisting of steps: treating the carbamate compound under acidic conditions to form an azaadamantane skeleton, deprotecting the azaadamantane compound and oxidizing the resulting compound.
(6) A catalyst for synthesizing organic compounds, which comprises an azaadamantan-N-oxyl compound of the formula (1) given above, or a derivative thereof.
(7) A catalyst as set forth above under (6), wherein the catalyst is for oxidizing organic compounds.
(8) A catalyst as set forth above under (6) or (7), wherein the organic compound is selected from alcohols.
(9) A method of oxidizing alcohols, which comprises the step of oxidizing an alcohol in the presence of an azaadamantan-N-oxyl compound of the formula (1) given above, or a derivative thereof, to form a corresponding oxo compound.
(10) An excellent, environment-conscious (environment friendly) alcohol oxidation catalyst that is an organic oxidation catalyst for oxidizing an alcohol, which comprises an effective amount of 1-methyl-2-azaadamantan-N-oxyl of the formula (4): 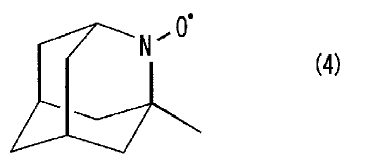 wherein an oxoammonium group is incorporated into the adamantane skeleton.
(11) A method of producing an excellent, environment-conscious (environment friendly) organic oxidation catalyst, said method being synthesis of said organic oxidation catalyst for oxidizing an alcohol, which comprises carrying out the first process consisting of steps: converting a bicyclo compound (7-methylenebicyclo[3.3.1]nonan-3-one) as a key starting material, obtainable via the Grob type ring opening reaction of 1,3-adamantanediol, to a corresponding oxime, then reducing the resultant oxime to a corresponding amine and further treating the amine with iodine to form a corresponding iodinated compound (1-iodomethyl-2-azaadamantane) and the second process consisting of steps: deiodinating the iodinated compound and then oxidizing the resulting compound to give 1-methyl-2-azaadamantan-N-oxyl serially.
(12) An azaadamantane compound of the formula (2): 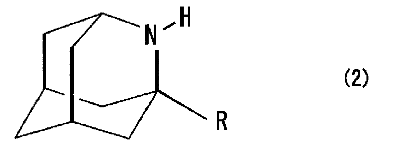 wherein R is an alkyl group, or a derivative thereof.

The present invention also provides techniques for producing, in a simple and easy manner and in high yields, an 1-alkyl-2-azaadamantan-2-oxyl compound, resulting from incorporation of a nitroxyl group into the adamantane skeleton, and serving as an organic oxidation catalyst for oxidizing alcohols. This invention provides mathods for producing a compound of the formula (1) given above, which comprises carrying out successively the first process consisting of steps: converting a key starting material, a bicyclo compound, as obtained via the Grob type ring opening reaction of 1,3-adamantanediol, to a corresponding oxime, then reducing the resultant oxime to a corresponding amine, treating the amine with carbobenzyloxy chloride to give a corresponding carbamate and treating the carbamate with 2N aqueous hydrochloric acid to form an azaadamantane compound and the second process consisting of steps: eliminating the carbamoyl group (deprotection) of the azaadamantane compound and oxidizing the resulting compound to give 1-methyl-2-azaadamantan-N-oxyl. The invention provides mathods for oxidizing organic compounds which comprises using an organic compound of the above formula (1) as an organic oxidation catalyst, and methods for oxidizing organic compounds which comprises using, as an oxidizing agent, an oxoammonium salt, prepared from a compound of the above formula (1) and chlorine.

### ADVANTAGEOUS PROFILES OF THE INVENTION

In accordance with the invention, not only alcohols having a relatively simple structure but also alcohols having a complicated steric configuration can be oxidized highly efficiently with a slight load (impact) on the environment, and the invention thus produces marked industrial effects. Further, according to the invention, such an effect is produced that, in the oxidation of alcohols relatively simple in structure, the products are obtained in high yields at lower catalyst usage levels as compared with the conventional catalysts. Further, according to the invention, such an effect is produced that the alcohol oxidation reaction, which is an essential chemical reaction in the manufacture of medicinal compounds, perfumes, liquid crystals and other organic functional substances, can be carried out highly efficiently while maintaining a high level of environment-friendliness.

The above objects and other objects, characteristic features, advantages and aspects of the present invention will become readily apparent to those skilled in the art from the following disclosures. It should be understood, however, that the disclosures given herein, including the following best modes of carrying out the invention, examples and others are illustrating preferred embodiments of the invention and given only for the purpose of illustration. It will be obvious to those skilled in the art that a great number of variations, and/or alterations (modifications) of this invention may be made without departing from the spirit and scope thereof as disclosed herein based on knowledge obtainable from the disclosure in the following parts and other parts of the present specification. All the patent documents and other references cited herein for illustrative purposes are hereby incorporated by reference into the present disclosure.

### BRIEF DESCRIPTION OF THE DRAWINGS

Fig. 1 is a scheme illustrating a preferred embodiment of the process for synthesizing 1-methyl-AZADO according to the invention.
Fig. 2 is a scheme illustrating the reaction mechanisms in which TEMPO, a conventional alcohol oxidation catalyst, is involved.
Fig. 3 is a scheme illustrating the decomposition of TEMPO.
Fig. 4 is a scheme illustrating a process for synthesizing a 1-alkyl-2-azaadamantan-N-oxyl according to the invention in a simple and easy manner and in high yields.

### BEST MODES OF CARRYING OUT THE INVENTION

The invention provides an azaadamantan-N-oxyl compound of the formula (1) given hereinabove, or a derivative thereof, which serves as a catalyst for synthesizing organic compounds, in particular as an organic catalyst and oxidation catalyst, intermediates for the synthesis thereof (e.g. an azaadamantane compound of the formula (2) given hereinabove, or a derivative thereof, a bicyclo[3.3.1]-nonanyl-3-amine compound of the formula (3) given hereinabove, or a derivative thereof, and an iodinated compound or carbamate compound derived therefrom) as well as techniques for synthesizing and utilizing the same.

For the substituents R and R^{a} herein, the "alkyl group" is not particularly limited to, but may be any of those which are known in the relevant field of art as long as they can achieve the intended object; including, for example, lower alkyl groups. The "lower alkyl group" includes an alkyl group containing 1-5 carbon atoms, such as methyl, ethyl, propyl, isopropyl, n-butyl, isobutyl, sec-butyl, tert-butyl, and pentyl. In particular, the preferred lower alkyl group is methyl.

For the substituent R^{a}, the "alkylidene group" is not particularly limited to, but may be any of those which are known in the relevant field of art as long as they can achieve the intended object; including, for example, lower alkylidene groups. The "lower alkylidene group" includes an alkylidene group containing 1-5 carbon atoms, such as methylidene, ethylidene, propylidene, isopropylidene (1-methylethylidene), butylidene, and sec-butylidene. In particular, the preferred lower alkylidene group is methylidene.

In the following, 1-alkyl-2-azaadamantan-N-oxyl compounds, which are representatives of the azaadamantan-N-oxyl compounds of the formula (1) given hereinabove or derivatives thereof, are described more specifically, taking 1-methyl-2-azaadamantan-N-oxyl (1-methyl-AZADO) as an example. It is obvious to those skilled in the art that the description can be adopted in the same manner to other compounds or derivatives.

The organic oxidation catalyst 1-methyl-AZADO according to the invention is an azaadamantan-N-oxyl type compound resulting from incorporation of a nitroxyl group into the adamantane skeleton, and the oxoammonium ion formed by oxidation of the nitroxyl group is a chemical species capable of rapidly oxidizing an alcohol to the corresponding aldehyde or ketone under mild conditions. The organic oxidation catalyst 1-methyl-AZADO according to the invention can form this oxoammonium ion stably on the adamantane skeleton.

1-Methyl-AZADO has a chemical structure represented by the following formula (4): 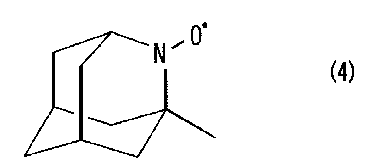 1-Methyl-AZADO occurs in the form of volatile red semicrystals showing a strong UV absorption at near 256 nm. As a result of incorporation of an oxoammonium group in the adamantane skeleton, the α-position hydrogen atom is stabilized by the Bredt's rule, the stability of the oxoammonium group is ensured, the steric hindrance is reduced as compared with TEMPO, and a wide range of reaction fields can thus be secured. Therefore, secondary alcohols having a sterically complicated structure, which can hardly be oxidized with TEMPO, can also be oxidized highly efficiently. Further, this compound is provided with a high level of chemical structure stability owing to the adamantane skeleton and the possibility of decomposition as found with TEMPO can be markedly reduced.

Now, a preferred synthesis of 1-methyl-AZADO is described as follows.

In the present invention, 1,3-adamantanediol is prepared by hydroxyl group introduction into 1-adamantanol (5), and this resulting 1,3-adamantanediol is subjected to the Grob type ring opening reaction to form a bicyclo compound (6) (7-methylenebicyclo[3.3.1]-nonan-3-one), which is preferably used as a key starting material. Typically, the procedure can be done according to the disclosure in Muraoka, O. et al., Syn. Commun., 26:1555 (1996). In the present invention, it is preferable to perform the first process consisting of steps: converting this bicyclo compound to the corresponding oxime, then reducing the resultant oxime to the corresponding amine and treating the amine with iodine to produce an iodinated compound (7) (1-iodomethyl-2-azaadamantane) and the second process consisting of steps: deiodinating the iodine compound and then oxidizing the resulting compound to produce 1-methyl-AZADO (4) serially. The reduction treatment to give the amine mentioned above can be carried out referring to, for example, Ipaktschi, J. et al., Chem. Ber., 117:856 (1984). The oxidation treatment for the above-mentioned N-oxyl formation can be carried out referring to, for example, Rychnovsky, et al., J. Org. Chem., 61:119 (1996). These steps are shown in Fig. 1.

The key material bicyclo compound is preferably prepared as follows:

To a solution of 1-adamantanol in CH₃CN-CCl₄-H₂C is added NaIO₄ and RuCl₃ successively and the mixture is reacted at 60°C for incorporation of a hydroxyl group into 1-adamantanol to give a diol (first reaction) and TsCl is then added to a solution of this diol in benzene-pyridine and the resultant mixture is reacted at 70°C (second reaction).

In the first process according to the invention, HONH₂·HCl is added to a solution of the above-mentioned key material bicyclo compound in pyridine to give the corresponding oxime (first reaction), MoO₃ is added to a solution of this oxime in MeOH, NaBH₄ is then added thereto with ice cooling and the resultant mixture is reacted at the same temperature for reduction to form the corresponding amine (second reaction), and I₂ is added to a solution of this amine in CH₃CN under protection against light and the resulting mixture is reacted at room temperature to give an iodinated compound (third reaction). In the first reaction, the use of HONH₂ or such a reagent as HONH₂-H₂SO₄, HONH₂-CH₃COOH or HONH₂-H₃PO₄ in lieu of HONH₂·HCl gives almost the same results. In the third reaction, N-iodosuccinimide may be used in lieu of I₂.

In the second process, LiAlH₄ is added to a solution of the iodinated compound (obtained in the first process) in THF at room temperature and the mixture is reacted with heating for deiodination to give the corresponding amine (first reaction). To a solution of this amine in H₂O-MeO is added Na₂WO₄-2H₂O at room temperature, and the mixture is stirred and then cooled with ice cooling, to which 30% H₂O₂ is added dropwise, or organic cooxidizer mCPBA or the like is added. After stirring, the temperature is raised to room temperature to allow the reaction to proceed to give 1-methyl-AZADO (second reaction). The use of various aluminum hydride reagents in lieu of LiAlH₄ used in the first reaction can also give equivalent results.

These processes are performed serially to afford 1-methyl-AZADO capable of serving as an organic oxidation catalyst excellent in environment-friendliness.

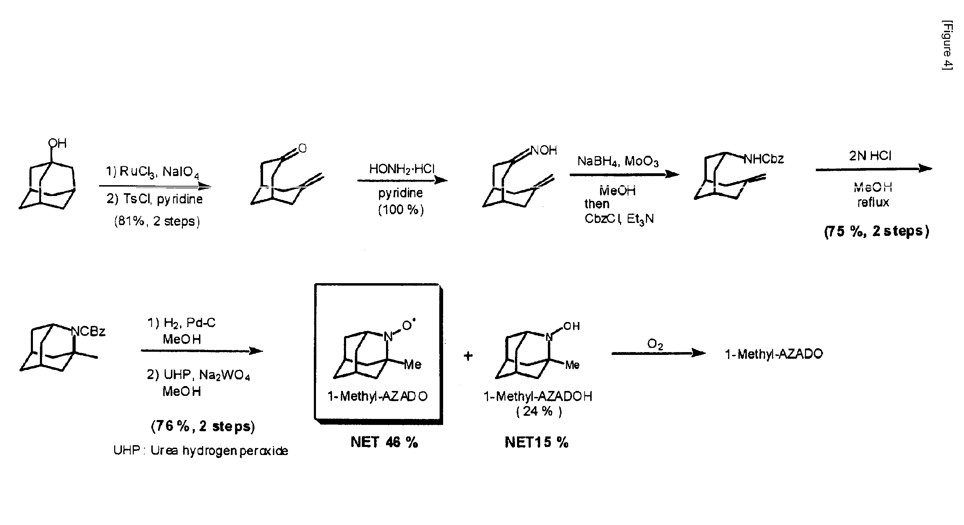

Fig. 4 is a chart illustrating the synthesis scheme of 1-methyl-2-azaadamantan-N-oxyl according to another embodiment of the invention. Described hereinbelow is this embodiment of the invention referring to the drawing.

In accordance with the invention, it is preferable that hydroxy group incorporation into 1-adamantanol gives 1,3-adamantanediol, which is then subjected to the Grob type cleavage reaction to form a bicyclo compound, preferably serving as the key starting material. In accordance with the invention, 1-methyl-2-azaadamantan-N-oxyl is produced by carrying out successively the first process consisting of steps in which this bicyclo compound is converted to the corresponding oxime, the resultant oxime is then reduced to produce the corresponding amine, the resulting amine is further treated with carbobenzyloxy chloride to form the carbamate compound and the carbamate compound is treated with an aqueous 2N hydrochloric acid solution to produce an azaadamantane compound and the second process consisting of steps in which the carbamoyl group of the azaadamantane compound is deprotected and the resulting compound is oxidized to give 1-methyl-2-azaadamantan-N-oxyl. It is evident that the above description can be construed as teaching the production of any other azaadamantan-N-oxyl compound of the formula (1) given hereinabove, or a derivative thereof, in lieu of 1-methyl-2-azaadamantan-N-oxyl (1-methyl AZADO).

The amine-protecting group as used herein is not limited to, but may include a benzyloxycarbonyl group as described in examples, as well as any amino-protecting group, without any particular limitation. The conditions for intramolecular ring closure to azaadamantane are not limited to, but include those in the presence of hydrochloric acid, as well as any protic acid or Lewis acid without any particular limitation.

In the synthetic process according to this embodiment, 1-methyl-2-azaadamantan-N-oxyl can be produced in 7 steps in an overall yield of 46%, for instance, as a result of improvements in process operability.

When used in a catalytic amount, the 1-alkyl-2-azaadamantan-N-oxyl, which is the organic nitroxyl radical of the invention, can convert primary and secondary alcohols to the corresponding carbonyl compounds with the combined use of an aqueous solution of sodium hypochlorite.

The oxidation reaction can be carried out for giving the azaadamantan-N-oxyl compound of the formula (1), or a derivative thereof, from the azaadamantane compound of the formula (2), or a derivative thereof, according to the above-described techniques and under the above-described conditions or according to the techniques and under the conditions, disclosed herein. For example, the oxidation is done by contacting with an oxidizing agent such as Na₂WO₄·2H₂O, H₂O₂, NaOCl or an organic cooxidizer and/or such various cooxidizers as those disclosed in Lidia D. L., et al.: J. Org. Chem. 2003, vol. 68, p. 4999 and Miller R. A., et al.: Org. Lett. 2003, vol. 53, p. 285, among others, in an appropriate solvent, for example an alcohol solvent such as anhydrous or hydrous methanol, ethanol, propanol or isopropanol. This oxidation can also be carried out by blowing an oxygen-containing gas, ozone or an active oxygen species into the reaction mixture.

The reaction can be carried out for obtaining the azaadamantane compound of the formula (2), or a derivative thereof, via intramolecular ring closing reaction of the bicyclo[3.3.1]nonanyl-3-amine compound of the formula (3), or a derivative thereof, for azaadamantane ring formation according to the above-described techniques and under the above-described conditions or according to the techniques and under the conditions, disclosed herein. For example, the reaction can be accomplished via treatment with an iodinating agent such as I₂ or N-iodosuccinimide, subsequent treatment of the thus-formed iodinated compound with a reducing agent, including an alkali metal aluminum hydride such as LiAlH₄, or by treatment with an amino-protecting group introducing agent, including a carbamating agent such as carbobenzyloxy chloride, followed by treatment with a protic acid such as hydrochloric acid, for instance.

The compound disclosed herein may encompass free forms thereof, and further salts thereof, hydrates thereof, solvates thereof, or any of derivatives derived from functional groups occurring in the compound molecule.
Among such compounds, some of the compounds may exist in more than one tautomeric form. This invention extends to all toautomeric forms. The compounds of the present invention may also contain one or plural asymmetric carbon atoms and thus give rise to optical isomers such as (R)-and (S)-isomers, racemates, diastereoisomers and others. The present invention includes all such possible isomers, and their racemic and resolved, enantiomerically pure forms, as well as all mixture thereof. The compounds disclosed herein can be isolated, in certain cases, as hydrates, solvates with, for example, ethanol and the like, and a variety of crystalline substances. The salts of the compounds may preferably include acceptable or usable nontoxic or low toxic inorganic acid and organic acid salts and acceptable or usable nontoxic or low toxic inorganic base and organic base salts. Examples of such salts include salts with halogen atom-derived anions (e.g. Cl⁻, Br⁻, I⁻, etc.), hydrochlorides, hydrobromides, sulfates, formates, acetates, propionates, fumarates, oxalates, maleates, citrates, succinates, tartrates, trifluoroacetates, methanesulfonates, benzenesulfonates, p-toluenesulfonates, etc.; alkali metal salts such as sodium salts and potassium salts, alkaline earth metal salts such as calcium salts and magnesium salts, aluminum salts, ammonium salts, methylamine salts, ethylamine salts, trimethylamine salts, triethylamine salts, aniline salts, pyridine salts, piperidine salts, picoline salts, ethanolamine salts, diethanolamine salts, triethanolamine salts, dicyclohexylamine salts, N,N'-dibenzylethylenediamine salts, etc.

The reactions described above can be carried out in the presence or absence of a solvent. When some reaction is carried out in the presence of a solvent, the solvent used can be selected from those conventional solvents which do not adversely affect the reaction. Said solvents include aromatic hydrocarbons, aliphatic hydrocarbons, esters, ethers, aliphatic halogenated hydrocarbons, alcohols, amides, organic acids and water, among others. The preferred solvent as used herein is ethanol, propanol, isopropanol, n-butanol, ethyl acetate, butyl acetate, formic acid, acetic acid, hexamethylphosphoramide, dimethyl-imidazolidinone, acetonitrile, N,N-dimethylformamide (DMF), dimethylacetamide, N-methylpiperidone, dimethyl sulfoxide (DMSO), pyridine, chloroform, dichloromethane, 1,2-dichloroethane, methylene chloride, dioxane, acetonitrile, toluene, benzene, xylene, hexane, pentane, heptane, tetrahydrofuran (THF), diethyl ether, diisopropyl ether, tert-butyl methyl ether, 1,2-dimethoxyethane, methylene chloride and the like. The solvent may be one of these or an appropriate mixture of two or more of them, may be anhydrous or hydrous, and an appropriate one is to be selected for use. The reaction temperature is ranging from about -80°C to about 200°C, preferably from room temperature to about 150°C. The reaction time can be selected for the desired reaction to be complete; generally, the reaction is carried out for about 1 hour to about 40 hours.

The amino-protecting group as can be used herein includes, for example, C₁₋₆ alkylcarbonyl (e.g. acetyl, propionyl, etc.), formyl, phenylcarbonyl, C₁₋₆ alkyloxycarbonyl (e.g. methoxycarbonyl, ethoxycarbonyl, tert-butoxycarbonyl, etc.), phenyloxycarbonyl (e.g. benzoxycarbonyl etc.), C₇₋₁₀ aralkyloxycarbonyl (e.g. benzyloxycarbonyl), trityl and phthaloyl, which may optionally have one or more substituents. The substituent(s) on these may include halogen atoms (e.g. fluorine, chlorine, bromine, iodine, etc.), C₁₋₆ alkylcarbonyl (e.g. acetyl, propionyl, butyryl, etc.) and nitro, among others, and the number of substituents is about 1 to 3. The carboxyl-protecting group as can be used herein includes, for example, C₁₋₆ alkyl (e.g. methyl, ethyl, propyl, isopropyl, butyl, tert-butyl, etc.), phenyl, trityl and silyl, which may optionally have one or more substituents. The substituent(s) on these may include halogen atoms (e.g. fluorine, chlorine, bromine, iodine, etc.), C₁₋₆ alkylcarbonyl (e.g. acetyl, propionyl, butyryl, etc.), formyl and nitro, among others, and the number of substituents is about 1 to 3. The methods of introducing and eliminating such protective groups include the methods known per se in the art or modifications thereof (e.g. the methods described in "Protective Groups in Organic Chemistry", J. F. W. McOmie et al., Plenum Press; "Protective Groups in Organic Synthesis", 3rd Edition (Theodora W. Greene, Peter G. M. Wuts, John Wiley & Sons, Inc., ISBN:0-471-16019-9, April 1999)) . The elimination includes methods comprising reduction, treatments with an acid or base, ultraviolet light, hydrazine, phenylhydrazine, sodium N-methyldithiocarbamate, tetrabutylammonium fluoride or palladium acetate, and others.

The reducing reagent may include, for example, sodium triacetoxyborohydride, sodium cyanoborohydride, sodium borohydride and others.

The compounds disclosed herein can be appropriately isolated and purified, according to need, by any of the separation/purification means known in the art, for example concentration, vacuum concentration, solvent extraction, crystallization, recrystallization, solvent replacement and/or chromatography.

The catalyst for synthesizing organic compounds according to the invention comprises at least an azaadamantan-N-oxyl compound of the formula (1) given hereinabove, or a derivative thereof, it is only required to contain a catalytically effective amount of the compound of formula (1), or a derivative thereof. In using it as a catalyst, the compound of formula (1), or a derivative thereof, may be added to a mixture containing at least one or more reactant starting materials. Alternatively, the reactant starting material(s) may be added to a solvent containing at least the compound of formula (1), or a derivative thereof. The proportion of the compound of formula (1), or a derivative thereof, to the starting material organic compound is not particularly limited to, so long as the desired level of catalytic activity can be obtained, but the compound of formula (1), or a derivative thereof, can be used, for example, in a mole ratio of 1/100,000 to 1/1, preferably 1/10,000 to 2/3, more preferably 1/1,000 to 1/10. The catalyst of the invention may be added to the reaction mixture in the form of a mixture of the compound of formula (1), or a derivative thereof, with such an oxidizing agent as an aqueous sodium hypochlorite solution. The catalyst is useful typically in the oxidation reaction of organic compounds. For example, the catalyst can be used in oxidizing an organic compound having at least a group sensitive to oxidation reaction. The group sensitive to oxidation reaction may include groups -OH, =O, etc. The organic compound may include, for example, those containing a hydroxyl group and/or a carbonyl group, among others, which can be found out by accessing the Chemical Abstracts database, for instance, and appropriate one(s) can be selected from those hit compounds. Typical organic compounds include, for example, alcohols, thiols, aldehydes, ketones, carboxylic acids and derivatives thereof (including acid halides, esters, etc.).

The alcohol may include, for example, primary alcohols having the formula: A-CH₂-OH and secondary alcohols having the formula: A-CH(OH)-B. They can be converted with an oxidizing agent, such as an aqueous solution of sodium hypochlorite, in the presence of said inventive catalyst to the corresponding carbonyl compounds. The oxidizing agent used herein includes those which can act in oxidizing the compound of the formula (2) given hereinabove, or a derivative thereof.

The above substituents A and B are not particularly limited to, but may be any of those organic groups which do not adversely affect the reaction; including, for example, an alkyl group, which may be unsubstituted or optionally substituted, a cycloalkyl group, which may be unsubstituted or optionally substituted, or an aromatic homocyclic or heterocyclic group, which may be unsubstituted or optionally substituted. The alkyl group in the "alkyl group, which may be unsubstituted or optionally substituted" as can be used herein for the substituents A and B includes C₁₋₆ alkyl groups, such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl and 3,3-dimethylpropyl. Here, the substituent(s) on said alkyl group may include lower alkoxy groups (e.g. C₁₋₆ alkoxy groups such as methoxy, ethoxy, and propoxy), halogen atoms (e.g. fluorine, chlorine, bromine, iodine, etc.), lower alkyl groups (e.g. C₁₋₆ alkyl groups such as methyl, ethyl, and propyl), lower alkenyl groups (e.g. C₂₋₆ alkenyl groups such as vinyl, and allyl), lower alkynyl groups (e.g. C₂₋₆ alkynyl groups such as ethynyl, and propargyl), an unsubstituted or optionally substituted amino group, an unsubstituted or optionally substituted hydroxyl group, an unsubstituted or optionally substituted sulfonyl group, an unsubstituted or optionally substituted sulfonylamino group, a cyano group, a nitro group, a nitroso group, an unsubstituted or optionally substituted amidino group, a carboxyl group, lower alkoxycarbonyl groups (e.g. C₁₋₆ alkoxycarbonyl groups such as methoxycarbonyl, and ethoxycarbonyl), an unsubstituted or optionally substituted carbamoyl group (e.g. a carbamoyl group, which may be unsubstituted or optionally substituted by a C₁₋₆ alkyl or acyl group (e.g. formyl, C₂₋₆ alkanoyl, benzoyl, nonhaloganated or optionally halogenated C₁₋₆ alkoxycarbonyl, nonhaloganated or optionally halogenated C₁-₆ alkylsulfonyl, benzenesulfonyl, etc.) wherein said C₁₋₆ alkyl or acyl group may be unsubstituted or optionally substituted by a 5- or 6-membered monocyclic aromatic heterocyclyl group (e.g. pyridinyl etc.), 1-aztidinylcarbonyl, 1-pyrrolidinylcarbonyl, piperidinocarbonyl, morpholinocarbonyl, 1-piperazinylcarbonyl, etc.), alkyl groups substituted by such an "optionally substituted cycloalkyl group" or "optionally substituted aromatic homocyclic or heterocyclic group" as will be mentioned hereinbelow, alkenyl groups substituted by such an "optionally substituted cycloalkyl group" or "optionally substituted aromatic homocyclic or heterocyclic group" as will be mentioned hereinbelow, alkoxy groups substituted by such an "optionally substituted cycloalkyl group" or "optionally substituted aromatic homocyclic or heterocyclic group" as will be mentioned hereinbelow, a hydroxyl group substituted by such an "optionally substituted cycloalkyl group" or "optionally substituted aromatic homocyclic or heterocyclic group" as will be mentioned hereinbelow, an amino group substituted by such an "optionally substituted cycloalkyl group" or "optionally substituted aromatic homocyclic or heterocyclic group" as will be mentioned hereinbelow, acyl groups substituted by such an "optionally substituted cycloalkyl group" or "optionally substituted aromatic homocyclic or heterocyclic group" as will be mentioned hereinbelow, and so forth, and one to three such optional substituents may be found at a site or sites allowing substitution.

The cycloalkyl group used in the "cycloalkyl group, which may optionally be substituted" for the substituents A and B includes, for example, C₃₋₇ cycloalkyl groups such as cyclopropyl, cyclobutyl, cyclopentyl, cyclohexyl and cycloheptyl. The substituent(s) on the cycloalkyl groups may include the same substituent species as mentioned above referring to the "alkyl group, which may be unsubstituted or optionally substituted", with the same number of said substituent species.

The aromatic homocyclic or heterocyclic group used in the "aromatic homocyclic or heterocyclic group, which may optionally be substituted" for the substituents A and B includes, for example, monocyclic or condensed polycyclic aromatic carbocyclic groups or monocyclic or condensed polycyclic heterocyclic groups. The preferable group used herein includes C₆₋₁₄ aromatic carbocyclic groups (aryl groups) or 5- to 14-membered aromatic heterocyclic groups (heteroaryl groups). The more preferred group includes C₆₋₁₀ aromatic carbocyclic groups (aryl groups) or 5- to 10-membered aromatic heterocyclic groups (heteroaryl groups) and still more preferably C₆ aromatic carbocyclic group (aryl group) or 5- or 6-membered aromatic heterocyclic groups (heteroaryl groups). Preferred specific examples of the "aromatic homocyclic group" are pentazol; C₆₋₁₄ aryl groups such as phenyl, naphthyl, anthryl, azulenyl, phenanthryl and acenaphthylenyl. Among them, phenyl, 1-napthyl, 2-naphthyl and the like are particularly preferred. The "aromatic heterocyclic group" includes, for example, aromatic heterocyclic groups each containing at least one hetero atom (preferably 1 to 4, more preferably 1 or 2 hetero atoms), wherein said hetero atom(s) may be one to three (preferably 1 or 2) hetero atom species selected from inter alia oxygen, sulfur and nitrogen atoms, as a ring system-constituting atom(s) (ring atom(s)). Representatives of the "aromatic heterocyclic group" are 5- or 6-membered monocyclic aromatic heterocyclic groups such as, for example, furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl as well as 8- to 12-membered condensed polycyclic aromatic heterocyclic groups such as, for example, benzofuranyl, isobenzofuranyl, benzo[b]thienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl. The preferable heterocyclic group used herein includes 5- or 6-membered monocyclic aromatic heterocyclic groups. The substituent(s) on the "aromatic homocyclic or heterocyclic group, which may optionally be substituted" may be protected, if necessary, in the conventional manner in organic chemical synthesis, without any particular limitation, unless the reaction is adversely affected; further, the protective group may be any one known in the relevant field of art.

This oxidation reaction can be carried out employing those conditions which are known in the relevant field of art. For example, the oxidation can be done via adding an oxidizing agent to a solution containing one or more reactant starting materials in the presence of an effective amount of the inventive catalyst, or adding an effective amount of the inventive catalyst to a solution containing one or more reactant starting materials followed by addition of an oxidizing agent. The oxidation reaction is generally carried out in a solvent. The solvent can be properly selected for use from among those mentioned hereinabove. The oxidation reaction conditions: reactant starting material species and amounts thereof, catalyst amounts, oxidizing agent species and amounts thereof, solvent species and amounts thereof, reaction time, reaction temperature and stirring, can be suitably selected according to specific targets. If necessary, optimum or more preferred conditions may be selected by carrying out experiments.

Details of the present invention are described by the following examples but such examples are provided only for illustrative purposes, and for referential embodiments of the present invention. These examples have been given herein for illustrating specific embodiments of the invention, but should not be construed as in any snse limiting the scope of the present invention disclosed herein. It should be understood in the present invention that various embodiments can be made or executed within the spirit, scope and concept disclosed herein. All the examples were carried out or can be carried out, unless otherwise disclosed herein specifically, by standard techniques which are well known and conventional to those skilled in the art.

### EXAMPLE 1

A starting material used herein was 1-adamantanol. To a solution (123 ml) of 1-adamantanol (50.4 mmol) in CH₃CN-CCl₄-H₂O (3:3:1 v/v) was added NaIO₄ (116 mmol) and RuC1₃ (0.5 mmol) stepwise, and the mixture was reacted with stirring at 60°C for 7 hours. The resultant reaction mixture was extracted and dried to give a crude diol product. Then, without further purification of this diol, TsCl (77 mmol) was added to a solution (150 ml) of the diol in benzene-pyridine (1:1 v/v), and the mixture was reacted with stirring at 70°C. After extraction and drying, the resultant residue afforded a bicyclo compound.

Then, the resultant bicyclo compound was used as a key starting material. Thus, HONH₂·HCl (50 mmol) was added to a pyridine solution (38 ml) of the bicyclo compound (25 mmol), and the mixture was reacted with stirring for 4 hours, then extracted, washed and dried. Thereafter, the resulting residue gave a corresponding oxime. To a MeOH solution (250 ml) of the oxime (25 mmol) obtained was added MoO₃ (30 mmol), and the mixture was stirred for 10 minutes. Thereafter, ice-cooled NaBH₄ (120 mmol) was added to the mixture, which was then reacted at the same temperature with stirring for 2 hours, then extracted and dried to give a crude amine product. To a CH₃CN solution of the resultant crude amine was added I₂ (25 mmol) while being protected from light, and the mixture was reacted with stirring at room temperature for 3 hours, then extracted, washed and dried. The resulting residue gave an iodinated compound.

To a THF solution (60 ml) of the resultant iodinated compound (12 mmol) was then added LiAlH₄ (14.5 mmol), and the mixture was heated under reflux for 30 minutes and, after ice cooling, a 30% NH₃ solution was added. After stirring, the reaction mixture was filtered, the resultant solid was dissolved and subjected to amine precipitation to give a crude crystalline amine. To a H₂O-MeOH solution (100 ml) of the resultant amine was added Na₂WO₄·2H₂O (1.2 mmol) at room temperature. After confirmation that the reaction mixture was turbid, the mixture was ice-cooled, and treated with 30% H₂O₂ (51 mmol) dropwise. After stirring, the temperature was elevated to room temperature, and the mixture was reacted with further stirring, and evaporated to give a residue, which gave 1-methyl-AZADO. This compound was subjected to mass spectrometry coupled with electron ionization under the following conditions: ion accelerating voltage, 3 kV; an ionization potential, 70 eV; and ionizing current, 300 µA, upon which it gave a molecular ion peak at m/z 166 and a base peak (100%) at m/z 93. In addition, it gave characteristic fragment ion peaks at m/z 79, 107, 134 and 149.

### EXAMPLE 2

To a solution of 1-adamantanol (20 g, 131 mmol) in CH₃CN-CCl₄-H₂O (120 ml, 3:3:1 v/v) was added stepwise NaI0₄ (67 g, 302 mmol) and RuC1₃ (540 mg, 1.3 mmol), and the resulting mixture was stirred vigorously at 60°C for 7 hours. To the mixture was then added stepwise 10% Na₂S₂O₃ and aqueous NaHCO₃, and the mixture was extracted with AcOEt. The organic layer was dried over MgSO₄ and evaporated under reduced pressure to give a crude 1,3-adamantanediol (2; 19.6 g). This compound was used in the next reaction step without purification. To a solution of the crude diol 2 in benzene-pyridine (200 ml, 1:1 v/v) was added p-TsCl (56 g, 290 mmol), and the mixture was stirred at 70°C. After confirming that the reaction was completed, H₂O was added and the resulting mixture was extracted with Et₂O. The organic layer was washed with brine, dried over MgSO₄, and evaporated under reduced pressure. The residue was subjected to silica gel column chromatography to give a white solid ketone, 7-methylenebicyclo[3.3.1]nonan-3-one (9.9 g, 66 mmol, 50%), obtained from the AcOEt-hexane (1:8 v/v) eluate fraction. Recrystallization of a portion thereof from petroleum ether gave colorless needles.

To a solution of 7-methylenebicyclo[3.3.1]nonan-3-one (3 g, 20 mmol) in pyridine (30 ml) was added HONH₂-HC1 (2.8 g, 40 mmol), and the mixture was stirred for 4 hours. The solvent was distilled off under reduced pressure, and H₂O was added to the residue, followed by extraction with AcOEt. The organic layer was washed with brine, dried over MgSO₄, and evaporated under reduced pressure. The residue was subjected to silica gel column chromatography to give a white solid oxime, 7-methylenebicyclo[3.3.1]nonan-3-one oxime (3.3 g, 20 mmol, 100%), obtained from the AcOEt-hexane (1:6 v/v) eluate fraction. Recrystallization of a portion thereof from petroleum ether gave colorless prisms.

To a solution of the thus-synthesized 7-methylenebicyclo[3.3.1]nonan-3-one oxime (5 g, 30.3 mmol) in methanol (300 ml) was added MoO₃ (6.5 g, 45.5 mmol), and the mixture was stirred for 10 minutes under ice cooling, then admixed with NaBH₄ (11.5 g, 303 mmol) portionwise, and stirred at the same temperature for 2 hours. To the mixture was added successively Et₃N (6.3 ml, 45.5 mmol) and CbzCl (6.5 ml, 45.5 mmol) under ice cooling after verifying the disappearance of the starting material with TLC (thin layer chromatography), and the resultant mixture was stirred at the same temperature for 1 hour. After verifying the completion of the reaction, (CH₃)₂CO was added to the mixture which was then stirred for 10 minutes, filtered through Celite, and evaporated under reduced pressure. To the residue was added H₂O and the mixture was extracted with AcOEt. The resultant organic layer was washed with brine, dried over MgSO₄, and evaporated under reduced pressure. The residue was subjected to silica gel column chromatography to give benzyl (7-methylene-bicyclo[3.3.1]non-3-yl)carbamate (6.5 g, 22.7 mmol, 75%) as a colorless oil, obtained from the AcOEt-hexane (1:16 v/v) eluate fraction.

To a solution of the above amine (10.8 g, 37.8 mmol) in methanol (38 ml) was added 2N HC1 (19 ml), and the mixture was heated under reflux for 1.5 hours. After completion of the reaction, the mixture was cooled to room temperature, and then evaporated under reduced pressure. To the residue was added H₂O and the mixture was extracted with AcOEt, the organic layer was washed with brine and dried over MgSO₄, and evaporated under reduced pressure. The residue was subjected to silica gel column chromatography to give N-benzyloxycarbonyl-1-methyl-2-azaadamantane (10.8 g, 37.8 mmol, 100%) as a colorless oil, obtained from the AcOEt-hexane (1:20 v/v) eluate fraction.

To a solution of N-benzyloxycarbonyl-1-methyl-2-azaadamantane (120 mg, 0.42 mmol) in methanol (4.2 ml) was added 10% Pd-C (12 mg), and the mixture was stirred in a H₂ atmosphere at room temperature for 2 hours. The reaction mixture was filtered through Celite, and evaporated under reduced pressure. To the residue was added aqueous Na₂CO₃, the mixture was extracted with CHC1₃, the organic layer was dried over K₂CO₃, and evaporated under reduced pressure to give a crude amine product (60 mg). This compound was used in the next reaction step without purification. To a solution of the crude amine in methanol (0.85 ml) was added Na₂WO₄·2H₂O (69 mg, 0.21 mmol), and the mixture was stirred for 30 minutes. After verifying the suspension state of the mixture, urea hydrogen peroxide (157 mg, 1.68 mmol) was added under ice cooling. After stirring for 1 hour, the temperature was elevated slowly to room temperature, and the mixture was then stirred for additional 1 hour. After completion of the reaction, the solvent was distilled off under reduced pressure. To the residue was added H₂O, the mixture was extracted with CHCl₃, the organic layer was washed with brine, then dried over K₂CO₃, and evaporated under reduced pressure. The residue was subjected to silica gel column chromatography to give a red semisolid nitroxyl radical, 1-methyl-2-azaadamantan-N-oxyl (53 mg, 0.32 mmol, 76%), obtained from the AcOEt-hexane (1:4 v/v) eluate fraction.

### EXAMPLE 3

Using 1-methyl-AZADO thus-synthesized, the activities thereof as an oxidation catalyst were first checked with the primary alcohols shown in Table 1. As for the reaction conditions, the catalyst was used in each amount specified in Table 1 in CH₂Cl₂, and KBr (0.1 eq.), n-Bu₄NBr (0.05 eq.) and NaCl (1.4 eq.) were further added, and the reaction was carried out under ice cooling. The reaction time was 20 minutes. After completion of the reaction, the percent yield of each product was determined. The percent yield was calculated by the formula: (actual yield, i.e., the amount of product)/(theoretical yield, i.e., calculated from the amount of consumed starting material) x 100 (%). For comparative examples, runs were carried out under the same reaction conditions using TEMPO, and each comparative yield was calculated. The results thus obtained are shown in Table 1.

**Table 1**

| | | | | |
|---|---|---|---|---|
| 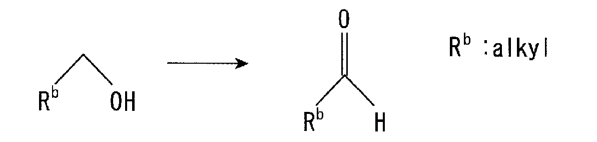 | | | | |

| Test No. | Alcohol species | Yield (%) | | |
|---|---|---|---|---|
| | | Catalyst | | |
| | | Equivalent | Me-AZADO (Invention) | TEMPO (Compar. Ex.) |
| 1-1 | 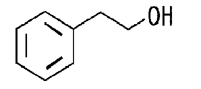 | 0.01 | 89 | 91 |
| 1-2 | 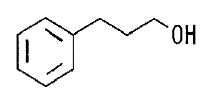 | 0.01 | 97 | 97 |
| 1-3 | | 0.001 | 95 | 96 |
| 1-4 | | 0.0001 | 65 (TON=6500) | 23 (TON=2300) |

Since, when the catalyst amount was 0.01 eq., 1-methyl-AZADO of the invention, as a primary alcohol oxidation catalyst, showed a function equivalent to that of the conventional TEMPO and, in addition, gave a higher yield in a reduced catalyst amount (0.0001 eq.) compared to the conventional TEMPO, with a higher catalyst turnover number (TON), it is evident that 1-methyl-AZADO of the invention has good performance characteristics as an excellent oxidation catalyst in oxidizing the primary hydroxyl group (primary alcohols).

Then, using 1-methyl-AZADO synthesized, the activities thereof as an oxidation catalyst were estimated in the same manner using various secondary alcohols specified in Tables 2 and 3. As for the reaction conditions, the catalyst amount was 0.01 eq. in CH₂Cl₂, and KBr (0.1 eq.), n-Bu₄NBr (0.05 eq.) and NaOCl (1.4 eq.) were further added, and the reaction was carried out under ice cooling. The reaction time was 20 minutes. After completion of the reaction, the percent yield of each product was determined. The percent yield was calculated by the formula: (actual yield, i.e., the amount of product)/(theoretical yield, i.e., calculated from the amount of consumed starting material) x 100 (%). For comparative examples, runs were carried out under the same reaction conditions using TEMPO, and each comparative yield was calculated. The results thus obtained are shown in Tables 2 and 3.

**Table 2**

| | | | |
|---|---|---|---|
| 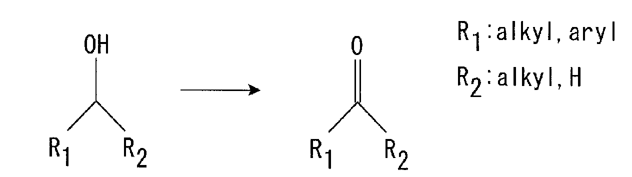 | | | |

| Test No. | Alcohol species | Yield (%) | |
|---|---|---|---|
| | | Catalyst | |
| | | Me-AZADO (Invention) | TEMPO (Compar. Ex.) |
| 2-1 | 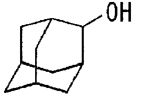 | 84 | 83 |
| 2-2 | 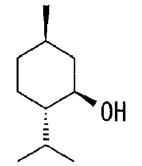 | 91 | 5 |
| 2-3 | 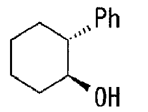 | 99 | 16 |
| 2-4 | 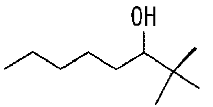 | 93 | 15 |
| 2-5 | 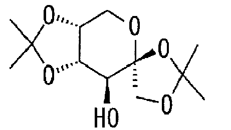 | 100 | 8 |
| 2-6 | 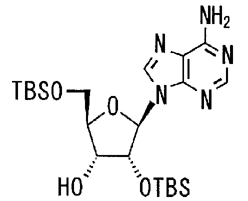 | 100 | 12 |

**Table 3**

| Test No. | Alcohol species | Yield (%) | |
|---|---|---|---|
| | | Catalyst | |
| | | Me-AZADO (Invention) | TEMPO (Compar. Ex.) |
| 2-7 | 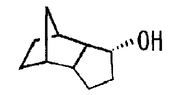 | 99 | 84 |
| 2-8 | 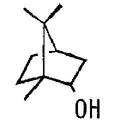 | 92 | 68 |
| 2-9 | 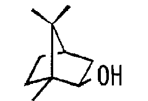 | 89 | 0 |
| 2-10 | 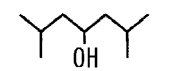 | 88 | 0 |
| 2-11 | 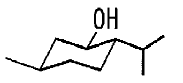 | 91 | 5 |

In the case of secondary alcohols having a relatively simple steric configuration (e.g. Test No. 2-1 and No. 2-7), the use of 1-methyl-AZADO of the invention as an oxidation catalyst and the use of TEMPO for comparison both gave target products in high yields. On the other hand, in the case of secondary alcohols having a sterically bulky, complicated structure, it was found that the use of 1-methyl-AZADO of the invention resulted in rapid oxidation, giving target products in high yields, whereas the use of TEMPO for comparison gave target products only in low yields.

In view of such results, it is evident that 1-methyl-AZADO is a catalyst useful as an oxidation catalyst not only for primary alcohols but also secondary alcohols.

### INDUSTRIAL APPLICABILITY

An excellent, environment-conscious organic oxidation catalyst for alcohols, capable of more efficient oxidation, as well as methods of producing the same can be utilized.

Use is made, as alcohol oxidation catalysts, of 1-alkyl-2-azaadamantan-N-oxyl species each synthesized from, as the key material, the bicyclo compound (obtained by the Grob type ring opening reaction of 1,3-adamantanediol), said bicyclo compound having a nitroxyl group incorporated in the adamantane skeleton. Since the inventive compounds have an oxoammonium group on the adamantane skeleton, the α-position hydrogen will be stabilized according to the Bredt's rule, the stability of the nitroxyl group will be ensured, the steric hindrance will be reduced as compared with the conventional oxidation catalyst TEMPO, and a wide reaction field will be secured. Therefore, they can highly efficiently oxidize not only primary alcohols but also those secondary alcohols having a sterically complicated structure which can hardly be oxidized with TEMPO.

The 1-alkyl-2-azaadamantan-N-oxyl species of the invention can be applied in synthesizing functional organic compounds, typically medicinal chemicals, perfumes and liquid crystals.

While the present invention has been described specifically in detail with reference to certain embodiments and examples thereof, it would be apparent that it is possible to practice it in other forms. In view of the above teachings, it will be understood that various modifications and variations are within the spirit and scope of the appended claims.

## Claims

1. An azaadamantan-N-oxyl compound of the formula (1): 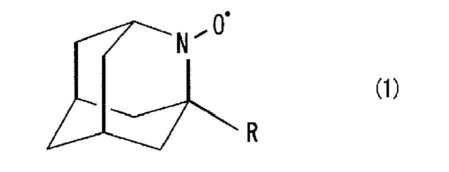 wherein R is an alkyl group, or a derivative thereof.

2. A method of producing an azaadamantan-N-oxyl compound of the formula (1): 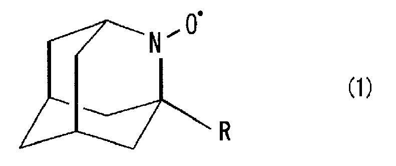 wherein R is an alkyl group, or a derivative thereof, which comprises at least the step of oxidizing an azaadamantane compound of the formula (2): 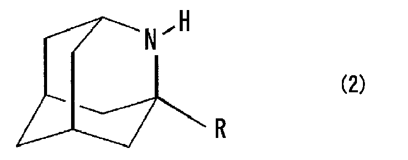 wherein R has the same meaning as defined above, or a derivative thereof.

3. A method of producing an azaadamantan-N-oxyl compound of the formula (1): 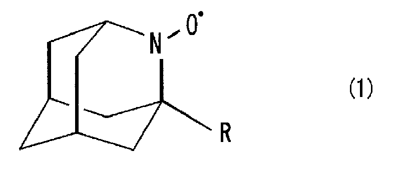 wherein R is an alkyl group, or a derivative thereof, which comprises at least the steps of
subjecting a bicyclo[3.3.1]nonanyl-3-amine compound of the formula (3): 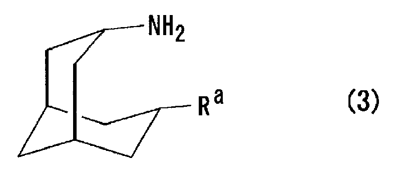 wherein R^{a} is an alkyl group or an alkylidene group bound to the bicyclo ring via a double bond, or a derivative thereof, to intramolecular ring closing reaction to form an azaadamantane ring and
oxidizing the resultant azaadamantane compound of the formula (2): 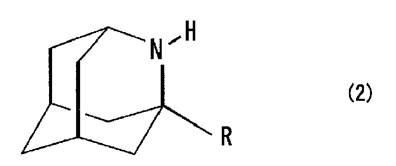 wherein R has the same meaning as defined above, or a derivative thereof.

4. A method of producing an azaadamantan-N-oxyl compound of the formula (1): 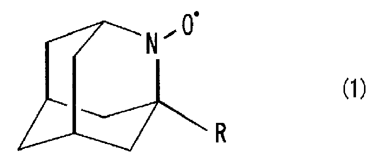 wherein R is an alkyl group, or a derivative thereof, which comprises at least a process selected from the group consisting of the first process consisting of steps: converting a bicyclo compound obtained by the Grob type ring opening reaction of 1,3-adamanatnediol to a corresponding oxime, reducing the resultant oxime to a corresponding amine, and further treating the amine with iodine to form a corresponding iodinated compound and the second process consisting of steps: deiodination of the iodinated compound, hydrogenation or alkylation and then oxidation of the resulting compound.

5. A method of producing an azaadamantan-N-oxyl compound of the formula (1): 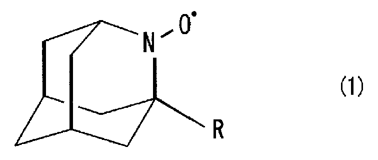 wherein R is an alkyl group, or a derivative thereof, which comprises at least a process selected from the group consisting of the first process consisting of steps: converting a bicyclo compound obtained by the Grob type ring opening reaction of 1,3-adamanatnediol to a corresponding oxime, reducing the resultant oxime to a corresponding amine, and further treating the amine with a carbamating agent to form a corresponding carbamate compound and the second process consisting of steps: treating the carbamate compound under acidic conditions to form an azaadamantane skeleton, deprotecting the azaadamantane compound and oxidizing the resulting compound.

6. A catalyst for synthesizing organic compounds, which comprises an azaadamantan-N-oxyl compound of the formula (1): 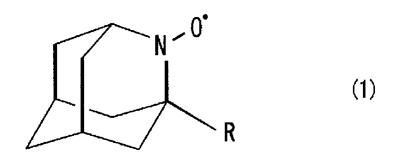 wherein R is an alkyl group, or a derivative thereof.

7. A catalyst according to Claim 6, wherein the catalyst is for oxidizing organic compounds.

8. A catalyst according to Claim 6 or 7, wherein the organic compound is selected from alcohols.

9. A method of oxidizing alcohols, which comprises the step of oxidizing an alcohol in the presence of an azaadamantan-N-oxyl compound of the formula (1): 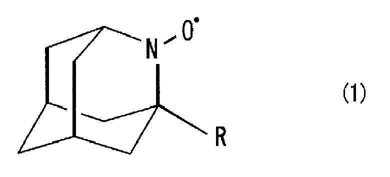 wherein R is an alkyl group, or a derivative thereof, to form a corresponding oxo compound.
